# EUROPEAN PATENT APPLICATION

(11) **EP 2 266 948 A1**
(43) Date of publication of application: **29.12.2010**
(21) Application number: 09384002.3
(22) Date of filing: 16.06.2009
(51) Int. Cl.: C07C 217/74, C07C 65/105, A61P 29/02, A61K 31/137, A61K 31/192

(54) **Salts of tramadol and diflunisal and their crystal form in the treatment of pain**

(71) Applicant: Laboratorios del. Dr. Esteve, S.A., 08041 Barcelona (ES)
(72) Inventor: Buschmann, Helmut Heinrich, 52076 Aachen (Walheim) (DE); Solà, Carandell, Lluis, 43005 Tarragona (ES); Céron, Betran, Jordi Carles, 43005 Tarragona (ES); Ramirez Artero, Jesus, 43130 Sant Ramon, Tarragona (ES)
(74) Representative: Peters, Hajo

(57) **Abstract**

The present invention relates to tramadol-diflunisal salts, processes for preparation of the same and their uses as medicaments or in pharmaceutical formulations, more particularly for the treatment of pain.

## Description

The present invention relates to salts of tramadol and diflunisal, processes for preparation of the same and their uses as medicaments or in pharmaceutical formulations, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

There are many drugs that are known to be useful in the treatment or management of pain. Opioids are frequently used as analgesics in pain. Derivatives of morphine are indicated for the treatment of moderate to acute pain in human. The analgesic effect is obtained through their action on morphinic receptors, preferably the µ-receptors. Among these derivatives of morphine may be mentioned morphine, codeine, pethidine, dextropropoxyphenemethadone, lenefopan and others.

One of the morphinic derivatives that has shown very good results when orally administrated, and which is now marketed is tramadol, also available as a physiologically acceptable salt, particularly as a chlorhydrate. Tramadol is a central acting analgesic drug that exerts its effects by activating opioid receptors and enhancing neuronal monoamine synaptic concentration. Tramadol whose chemical name is 2-(dimethylaminomethyl)-1-(3-methoxyphenyl)cyclohexanol, has the following formula :

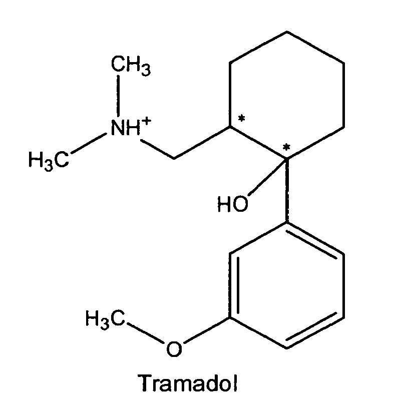

This structure shows two different chiral centers and thus may exist in two different diastereoisomers among which the tramadol is the cis-diastereoisomer: (1R, 2R), or (1S, 2S), both also known as (+)-tramadol and (-)-tramadol and both of which contribute in different ways to its activity. From the art it appears that this compound is neither fully opioid-like, nor non-opioid-like. Some studies have demonstrate that tramadol is an opioid agonist, whereas clinical experience indicates that it lacks many of the typical side effects of an opioid agonist, for example respiratory depression, constipation or tolerance.

Due to their drawbacks, opioids as analgesics to treat pain cannot always be given repeatedly or at higher doses. As a source to review the side effects of opioids that are known in the art one can mention e.g. J. Jaffe in "Goodman and Gilman's, The Pharmacological Basis of Therapeutics", 8th edition; Gilman et al.; Pergamon Press, New York, 1990, Chapter 22, pages 522-573.

Consequently, it has been proposed to combine opioids with other drugs that are not opioid analgesic agents in order to lower the amount of opioids needed to produce an equivalent degree of analgesia. Among these combinations, the association of tramadol with nonsteroidal anti-inflammatory drugs (NSAIDs) has been reported to be of particular interest (EP-0 546 676). US 5,516,803 (belonging to the patent family of EP-0 546 676) discloses a combination of tramadol with nonsteroidal antiinflammatory drugs (NSAIDs), specifically ibuprofen and discloses that the combination of tramadol-HCl with non-steroidal antiinflammatories, such as for example Ibuprofen, in a composition ratio of 1:1 to 1:200 produces a synergistically enhanced analgesic action and reduces the undesired accompanying symptoms.

US 6,558,701 patent discloses combination of tramadol with diclofenac stating that "for the treatment of moderate to severe pain, the World Health Organization (WHO) recommends combining opioid analgesics with non-steroidal analgesics in order to produce a more effective pain relief and possibly reduce amounts of analgesic which are necessary to administer".

One interesting NSAIDs to be combined with tramadol is the marketed drug diflunisal, whose chemical name is 5-(2,4-difluorophenyl)-2-hydroxy-benzoic acid. Diflunisal is a well-known analgesic mostly used to treat arthritis. Diflunisal shows the following structure:

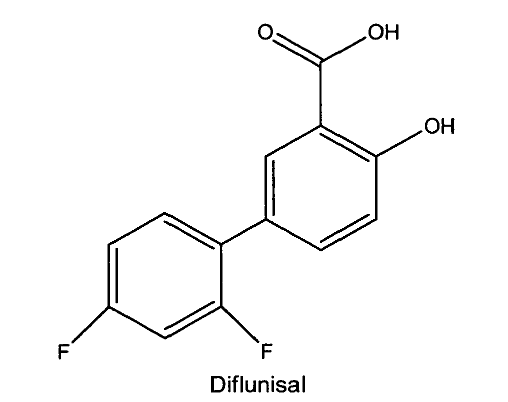

The applicant has now found that tramadol and diflunisal can be combined to form a mixed-salt.

Thus the object of the present invention is a salt of tramadol and diflunisal of this formula:

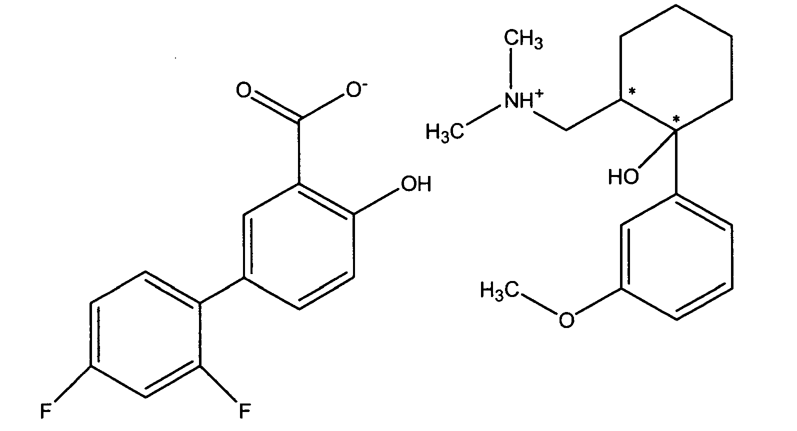

In general, each active principle forming part of the salts according to the invention, tramadol and diflunisal, has its own disadvantages when used alone or in the form of the salts known from the art.

Tramadol hydrochloride, which is often used orally, displays a highly bitter taste, which makes the drug often difficult to swallow and lowers patient compliance. Also - as stated before - the drawbacks associated with opioids is limiting their use, so that they have to be given at lower doses and often less frequently as their use as analgesics to treat pain would normally require.

On the other hand diflunisal is well-known to be - according to the Martindale - practically insoluble in water, which is limiting its use. In its free acid form that is used in the marketed formulations it only properly dissolves in slightly basic media.

As in addition it is well-known that often there are a number of chemical difficulties to be overcome for obtaining salts of diflunisal or tramadol, there still is a clear need for new drugable forms of tramadol or diflunisal.

These should either
- be active in pain or even more active when compared to tramadol base or hydrochloride salt or diflunisal; or
- be easily obtainable, or
- be easily crystallized, allowing more flexibility in formulating, or
- be highly soluble, especially if compared to diflunisal, allowing better dissolution rates, especially if dissolving in an aqueous physiological surrounding, or
- have as acidic partner of the tramadol a molecule having a beneficial pharmacological effect in itself, thus allowing for a highly efficient dose/weight relation of the active principle; or
- allow for a lower amount of tramadol being used to achive the same analgesic effect;
   or
- mask the bitter taste of tramadol, improving patience compliance on oral use; or
- have as basic partner of the diflunisal a molecule having a beneficial pharmacological effect in itself, thus allowing for a highly efficient dose/weight relation of the active principle.

Most desirably the new drugable form should combine more than one, most preferably all of these advantages.

Especially desirable improvements/advantages of the new drugable form would include:
- improvement of physicochemical properties in order to facilitate the formulation, the manufacture, or to enhance the absorption and/or the bioavailability; or
- being more active, especially as analgesic, when compared to tramadol base or its hydrochloride salt; or
- providing a new form of tramadol (with diflunisal having a beneficial pharmacological effect in itself), thus allowing for a highly efficient dose/weight relation of the final active principle; or
- allowing the use of a lower therapeutic dose of either tramadol and diflunisal or of both; or
- having a synergistic effect through the combination of tramadol and diflunisal in the same new drugable form; or
- being easily obtainable, easy to manufacture; or
- allowing more flexibility in formulating, or facilitating its formulation; or
- being highly soluble, thus allowing better dissolution rates, especially if dissolving in an aqueous physiological surrounding; or
- improve stability of the salt in comparison with the physical mixture of tramadol and diflunisal; or
- allowing new routes of administration; or
- allowing to combine tramadol with a chemically usually non-compatible active agent in the same formulation or even in immediate contact, without having to isolate tramadol minimizing/reducing the side effects, especially the severe side effects, assigned to tramadol.

Further, the new drugable form of tramadol should desirably show at least one, preferably more, of the following features:
- to have a very small particle size, e.g. from 300 µm or lower; or
- to be and/or remain essentially free of agglomerates; or
- to be less or not very hygroscopic; or
- to allow by selection of the counter-ion of tramadol to help in formulating controlled release or immediate release formulations; or
- to have a high chemical stability; or
   if given to a patient
- to decrease the inter- and intra-subject variability in blood levels; or
- to show a good absorption rate (e.g. increases in plasma levels or AUC); or
- to show a high maximum plasma concentration (e.g. Cₘₐₓ); or
- to show decreased time to peak drug concentrations in plasma (tₘₐₓ); or
- to show changes in half life of the compound (t_{1/2}), in whichever direction this change is preferably directed.

The applicant has now found that tramadol and diflunisal can be combined to form a well-soluble mixed-salt. Thus the abovementioned objective was achieved by providing these new salts of tramadol with diflunisal as these salts show improved properties if compared to tramadol and/or diflunisal alone.

Accordingly, the invention is drawn to a salt of tramadol with diflunisal.

This association of the two active principles into the same salt exhibits several advantages. Being linked as ion and counter-ion, they behave as a single chemical entity, thus facilitating the treatments, formulation, dosage etc. Further the physico-chemical properties are also improved. The formulation of the association is even easier with a solid to manipulate and an enhanced stability. The solubility may be also enhanced. Further, the combination of the two active principles into one unique species may advantageously remove the bitter taste of the tramadol hydrochloride, which makes oral application to a patient much easier. Another advantage is that the combination of the two active principles into one unique species seems to allow for a better Pharmacokinetic/Pharmacodynamic (PKPD) which could help in the treatment of pain. Thus, the mixed salts according to the invention do combine a high number of advantages over the state of the art.

In general, in most embodiments in which the salts of tramadol are used (e.g. for the treatment of pain etc.), these salts would be formulated into a convenient pharmaceutical formulation or a medicament. Accordingly a desirable advantage of a tramadol salt, especially if crystallized, would show improved pharmaceutical properties and features, especially when compared to the free base or tramadol hydrochloride.

In one preferred embodiment of the salt according to the invention the salt is formed of (rac)-tramadol with diflunisal.

In another preferred embodiment of the salt according to the invention the salt is formed of (+)-tramadol with diflunisal.

In another preferred embodiment of the salt according to the invention the salt is formed of (-)-tramadol with diflunisal.

The applicant has further demonstrated the possibility to crystallize said salts. Even though amorphous salts are also an aspect of the current invention, most preferred are crystalline salts. By that way the physico-chemical properties are improved. The formulation of the mixed salt is even easier with a solid to manipulate and an enhanced stability. The solubility, in particular the solubility of the diflunisal is also greatly augmented.

As the applicant has shown the possibility to crystallize said salts according to the invention a crystalline form of a salt according to the invention, is a separate, highly interesting aspect of the current invention.

Accordingly, a further aspect of the invention relates to a crystalline form of a salt according to the invention described above.

In a further preferred embodiment of the crystalline form of a salt according to the invention - especially of one salt being a salt of (rac)-tramadol with diflunisal - the crystalline form shows a Powder X-Ray Diffraction pattern with peaks [2θ] at 4.1, 7.2, 10.8, 11.1, 12.1, 12.9, 14.2, 14.4, 14.7, 16.1, 17.1, 17.7, 18.3, 18.5, 19.0, 19.3, 20.4, 21.1, 21.2, 21.7, 22.3, 22.4, 22.7, 23.0, 23.8, 24.0,24.4, 25.3, 26.1, 26.3, 26.9, 27.7, 28.7, 29.1, 30.4 and 31.3 [°].
The 2θ values were obtained using copper radiation (Cu_{Kα1} 1.54060Å).

The invention also concerns the crystalline form of a salt according to the invention - especially of one salt being a salt of (rac)-tramadol with diflunisal - the crystalline form showing an X-Ray powder diffraction spectrum with peaks expressed in d-Value in A at 21.38, 12.29, 8.17, 7.97, 7.31, 6.86, 6.22, 6.14, 6.01, 5.49, 5.19, 5.02, 4.84, 4.79, 4.67, 4.59, 4.34, 4.21, 4.18, 4.09, 3.99, 3.96, 3.92, 3.87, 3.73, 3.70, 3.65, 3.52, 3.41, 3.38, 3.31, 3.22, 3.10, 3.06, 2.94, and 2.85.

In a further preferred embodiment of the crystalline form of a salt according to the invention - especially of one salt being a salt of (rac)-tramadol with diflunisal - the crystalline form having an endothermic peak corresponding to the melting point with an onset at 160°C.

In a further preferred embodiment of the crystalline form of a salt according to the invention - especially of one salt being a salt of (+)-tramadol with diflunisal of crystalline form "A" - the crystalline form shows a Powder X-Ray Diffraction pattern with peaks [2θ] at 8.0, 9.4, 10.6, 11.5, 12.4, 13.3, 14.1, 15.4, 15.7, 16.3, 16.5, 17.5, 18.2, 18.4, 19.0, 19.7, 20.1, 20.5, 21.0, 21.4, 21.7, 22.2, 22.9, 23.2, 23.6, 24.2, 24.4, 24.9, 25.4 and 25.6 [°].
The 2θ values were obtained using copper radiation (Cu_{Kα1} 1.54060Å).

The invention also concerns the crystalline form of a salt according to the invention - especially of one salt being a salt of (+)-tramadol with diflunisal of crystalline form "A" - the crystalline form showing an X-Ray powder diffraction spectrum with peaks expressed in d-Value in A at 10.99, 9.39, 8.38, 7.67, 7.15, 6.66, 6.29, 5.76, 5.66, 5.43, 5.36, 5.07, 4.87, 4.81, 4.68, 4.50, 4.41, 4.32, 4.23, 4.15, 4.09, 4.01, 3.88, 3.82, 3.76, 3.68, 3.65, 3.57, 3.51, and 3.48.

In a further preferred embodiment of the crystalline form of a salt according to the invention-especially of one salt being a salt of (+)-tramadol with diflunisal of crystalline form "A" - the crystalline form having an endothermic peak corresponding to the melting point with an onset at 135°C.

In a further preferred embodiment of the crystalline form of a salt according to the invention - especially of one salt being a salt of (+)-tramadol with diflunisal of crystalline form "B" - the crystalline form shows a Powder X-Ray Diffraction pattern with peaks [2θ] at 10.8, 12.7, 12.8, 13.3, 15.6, 15.9, 17.2, 17.8, 18.4, 18.8, 19.8, 20.7, 21.4, 21.7, 21.8, 22.4, 24.9, 25.3, 25.6, 25.8, 26.1, 26.2, 27.0, 27.5, 28.4, 28.5, and 28.9 [°].
The 2θ values were obtained using copper radiation (Cu_{Kα1} 1.54060Å).

The invention also concerns the crystalline form of a salt according to the invention - especially of one salt being a salt of (+)-tramadol with diflunisal of crystalline form "B" - the crystalline form showing an X-Ray powder diffraction spectrum with peaks expressed in d-Value in A at 8.22, 6.95, 6.90, 6.64, 5.68, 5.57, 5.14, 4.98, 4.81, 4.72, 4.48, 4.29, 4.16, 4.10, 4.07, 3.97, 3.57, 3.52, 3.47, 3.45, 3.41, 3.40, 3.30, 3.24, 3.14, 3.13, and 3.09.

In a further preferred embodiment of the crystalline form of a salt according to the invention-especially of one salt being a salt of (+)-tramadol with diflunisal of crystalline form "B" - the crystalline form having an endothermic peak corresponding to the melting point with an onset at 121 °C.

In a further preferred embodiment of the crystalline form of a salt according to the invention - especially of one salt being a salt of (-)-tramadol with diflunisal of crystalline form "A" - the crystalline form shows a Powder X-Ray Diffraction pattern with peaks [2θ] at 7.8, 8.1, 9.4, 10.6, 11.5, 12.4, 13.3, 14.1, 15.4, 15.7, 16.3, 16.5, 17.0, 17.5, 18.2, 18.4, 19.0, 19.7, 20.1, 20.6, 21.0, 21.4, 21.7, 22.1, 22.9, 23.2, 23.6, 24.4, 24.9 and 25.4 [°].
The 2θ values were obtained using copper radiation (Cu_{Kα1}, 1.54060Å).

The invention also concerns the crystalline form of a salt according to the invention - especially of one salt being a salt of (-)-tramadol with diflunisal of crystalline form "A" - the crystalline form showing an X-Ray powder diffraction spectrum with peaks expressed in d-Value in A at 11.34, 10.97, 9.39, 8.36, 7.66, 7.15, 6.66, 6.29, 5.75, 5.65, 5.42, 5.36, 5.21, 5.07, 4.86, 4.81, 4.68, 4.50, 4.41, 4.31, 4.23, 4.15, 4.09, 4.01, 3.88, 3.82, 3.76, 3.65, 3.57, and 3.51.

In a further preferred embodiment of the crystalline form of a salt according to the invention-especially of one salt being a salt of (-)-tramadol with diflunisal of crystalline form "A" - the crystalline form having an endothermic peak corresponding to the melting point with an onset at 135°C.

In a further preferred embodiment of the crystalline form of a salt according to the invention - especially of one salt being a salt of (-)-tramadol with diflunisal of crystalline form "B" - the crystalline form shows a Powder X-Ray Diffraction pattern with peaks [20] at 10.7, 12.7, 13.3, 15.6, 15.9, 17.2, 17.8, 18.4, 18.8, 19.8, 20.7, 21.0, 21.3, 21.6, 21.9, 22.4, 23.5, 24.0, 25.3, 25.6, 25.8, 26.2, 27.0, 27.5, 28.0, 28.4, 28.5, 28.9, 29.2, and 32.2 [°].
The 2θ values were obtained using copper radiation (Cu_{Kα1}, 1.54060Å).

The invention also concerns the crystalline form of a salt according to the invention - especially of one salt being a salt of (-)-tramadol with diflunisal of crystalline form "B" - the crystalline form showing an X-Ray powder diffraction spectrum with peaks expressed in d-Value in A at 8.22, 6.95, 6.64, 5.68, 5.58, 5.14, 4.98, 4.81, 4.72, 4.48, 4.28, 4.23, 4.16, 4.10, 4.06, 3.97, 3.78, 3.70, 3.52, 3.47, 3.45, 3.40, 3.30, 3.24, 3.19, 3.14, 3.13, 3.09, 3.06, and 2.78.

In a further preferred embodiment of the crystalline form of a salt according to the invention-especially of one salt being a salt of (-)-tramadol with diflunisal of crystalline form "B" - the crystalline form having an endothermic peak corresponding to the melting point with an onset at 121 °C.

Another embodiment of the present invention relates to a process for the production of a salt according to the invention as described above comprising the steps of:
a) dissolving diflunisal as a free acid in an organic solvent and either together, before or after dissolving tramadol either as a free base or as a salt in an organic solvent, leading to a mixture in which both active principles are dissolved in one or more organic solvents;
b) evaporating the solvent to dryness,
   and either
c1) forming a slurry of the residue of step b) in a second organic solvent;
e1) filtering, drying and/or purifying the resulting product;
   or
c2) dissolving the residue of step b) in a second organic solvent:
d2) evaporating the second organic solvent at low temperature;
e2) filtering, drying and/or purifying the resulting product.

Preferably in the process above
- the organic solvent of step a) is methanol; and/or
- the second organic solvent of step c1) is diisopropyl ether; and/or
- the second organic solvent of step c2) is selected from acetone, ethanol, THF, ethyl acetate, chloroform; preferably acetone; and/or
- the temperature for evaporating the second organic solvent in step d2) is -18°C; and/or
- the ratio of tramadol to diflunisal is 2:1 to 1:2.

Both parts of the salt are well-known drugs used for a long time worldwide. Due to the therapeutic interest in tramadol in the treatment of pain symptoms like diabetic neuropathy and the well-known properties of COX-INHIBITORS like diflunisal in this field of medical indication, a further object of the present invention is a medicament containing a tramadol-diflunisal salt, or its crystalline form according to the invention.

Thus the invention also concerns a medicament comprising at least one salt according to the invention as described above and optionally one or more pharmaceutically acceptable excipients.

A further object of the invention is a pharmaceutical composition **characterized in that** it comprises an efficient amount of the crystalline form of at least one salt according to the invention as described above, in a physiologically acceptable medium.

The medicament according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modem Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more of the salts or their crystalline form as defined herein and 40-99 % by weight of one or more auxiliary substances (additives/excipients).

The compositions of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance to be administered during one or several intakes per day.

A further aspect of the invention relates to the use of a salt according to the invention as described above or of a crystalline form of at least one salt according to the invention as described above for the treatment of pain, preferably acute pain, chronic pain, neuropathic pain, hyperalgesia, allodynia or cancer pain, including diabetic neuropathy or osteoarthritis. A related further aspect of the invention is aimed at the use of a salt according to the invention as described above or of a crystalline form of at least one salt according to the invention as described above in the manufacture of a medicament for the treatment of pain, preferably acute pain, chronic pain, neuropathic pain, hyperalgesia, allodynia or cancer pain, including diabetic neuropathy or osteoarthritis. Preferably this use is provided for in form of a medicament or a pharmaceutical composition according to the invention as described above.

Another object of the current invention is a method of treatment of pain, preferably acute pain, chronic pain, neuropathic pain, hyperalgesia, allodynia or cancer pain, including diabetic neuropathy or osteoarthritis, by providing to a patient in need thereof a sufficient amount of a salt according to the invention as described above or of a crystalline form of at least one salt according to the invention as described above. Preferably a salt according to the invention or its crystalline form according to the invention is provided in physiologically suitable form like e.g. in form of a medicament or a pharmaceutical composition according to the invention as described above.

The present invention is illustrated below with the help of the following figures and examples. These illustrations are given solely by way of example and do not limit the invention.

### Brief description of the figures:

**Figure 1****:** DSC and TG analysis of (+)-tramadol-diflunisal crystalline salt form "A".
**Figure 2****:** Powder X-Ray diffraction pattern of (+)-tramadol-diflunisal crystalline salt form "A".
**Figure 3****:** DSC and TG analysis of (+)-tramadol-diflunisal crystalline salt form "B".
**Figure 4****:** Powder X-Ray diffraction pattern of (+)-tramadol-diflunisal crystalline salt form "B".
**Figure 5****:** DSC and TG analysis of (-)-tramadol-diflunisal crystalline salt form "A".
**Figure 6****:** Powder X-Ray diffraction pattern of (-)-tramadol-diflunisal crystalline salt form "A".
**Figure 7****:** DSC and TG analysis of (-)-tramadol-diflunisal crystalline salt form "B".
**Figure 8****:** Powder X-Ray diffraction pattern of (-)-tramadol-diflunisal crystalline salt form "B".
**Figure 9****:** DSC and TG analysis of (rac)-tramadol-diflunisal salt
**Figure 10****:** Powder X-Ray diffraction pattern of (rac)-tramadol-diflunisal salt.

### EXAMPLE

### Example 1: (+)-tramadol-diflunisal crystaline salt form "A".

### Preparation

A solution of diflunisal (20 mg, 0.08 mmol, 1 eq) in 0.5 mL of methanol was added to a solution of (+)-tramadol (21 mg, 0.08 mmol, 1 eq) in 0.5 mL of methanol. The mixture was evaporated to dryness, and the residue obtained was dissolved in acetone (0.3 mL). The resulting solution was evaporated at low temperature (-18°C). After two weeks, a solid was formed, which was dried under vacuum (10 mm Hg) at 40°C for 6 hours to give an abundant white solid corresponding to the salt (+)-tramadol / diflunisal of crystalline form "A".

The (+)-tramadol / diflunisal crystalline salt form "A" has been obtained starting with a ratio of (+)-tramadol / diflunisal of 1:1. The salt has been also obtained by evaporation at low temperature in chloroform.

### Characterization of (+)-tramadol-diflunisal crystalline salt form "A":

### ¹H-NMR spectrum of the (+)-tramadol-diflunisal crystalline salt form "A"

Proton nuclear magnetic resonance analyses were recorded in deuterated methanol (MeOH-d4) in a Bruker Avance 400 Ultrashield NMR spectrometer. Spectra were acquired solving 2 - 10 mg of sample in 0.4 - 0.8 mL of deuterated solvent.
**¹H NMR** (400 MHz, d4-methanol) δ: 1.45-2 (m, 8H), 2.22 (m, 1H), 2.62 (m, 1H), 2.64 (s, 6H), 2.95 (dd, *J* = 9 Hz, *J* = 13 Hz, 1H), 3.81 (s, 3H), 6.85 (m, 2H), 6.95-7.15 (m, 4H), 7.30 (t, *J* = 8 Hz, 1H), 7.47 (m, 2H), 8.01 (s, 1H).

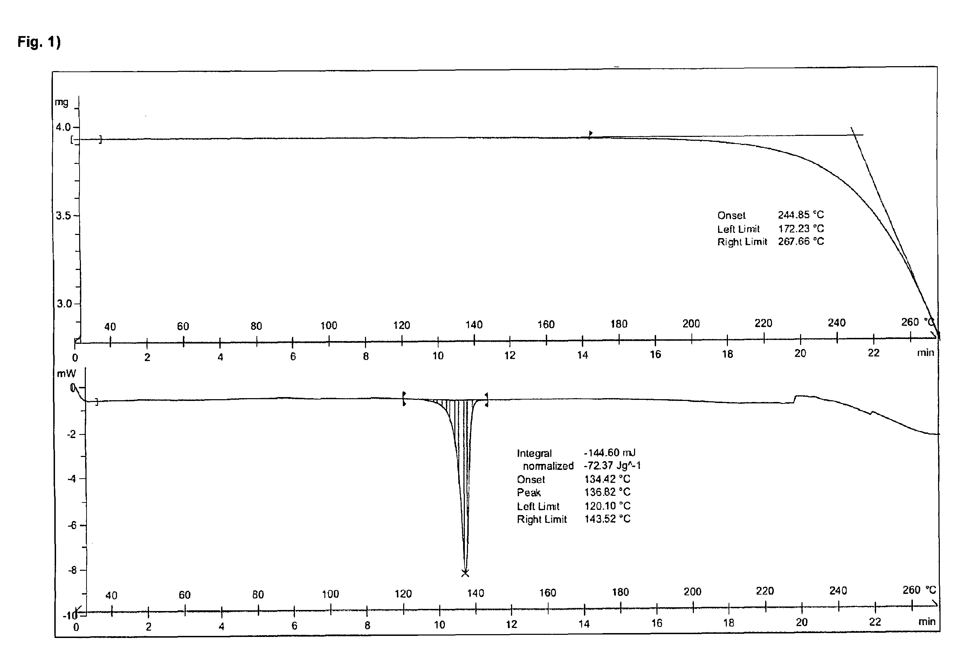

### DSC analysis of the (+)-tramadol-diflunisal crystalline salt form "A" (see Fig. 1)

DSC analyses were recorded in a Mettler Toledo DSC822e. Samples of 1-2 mg were weighted into 40 µL aluminium crucibles with a pinhole lid, and were heated, under nitrogen (50 mL/min), at 10°C/min from 30 to 300°C.
DSC (10°C/min): Endothermic peak corresponding to the melting point with an onset at 134°C.

### TG analysis of the (+)-tramadol-diflunisal crystalline salt form "A" (see Fig. 1)

Thermogravimetric analyses were recorded in a Mettler Toledo SDTA851e. Samples of 3 - 4 mg were weighted into 40 µL aluminium crucibles with a pinhole lid, and heated at 10°C/min from 30 to 600°C, under nitrogen (80 mL/min).
**TG** (10°C/min): No weight loss observed at temperatures below the melting point.

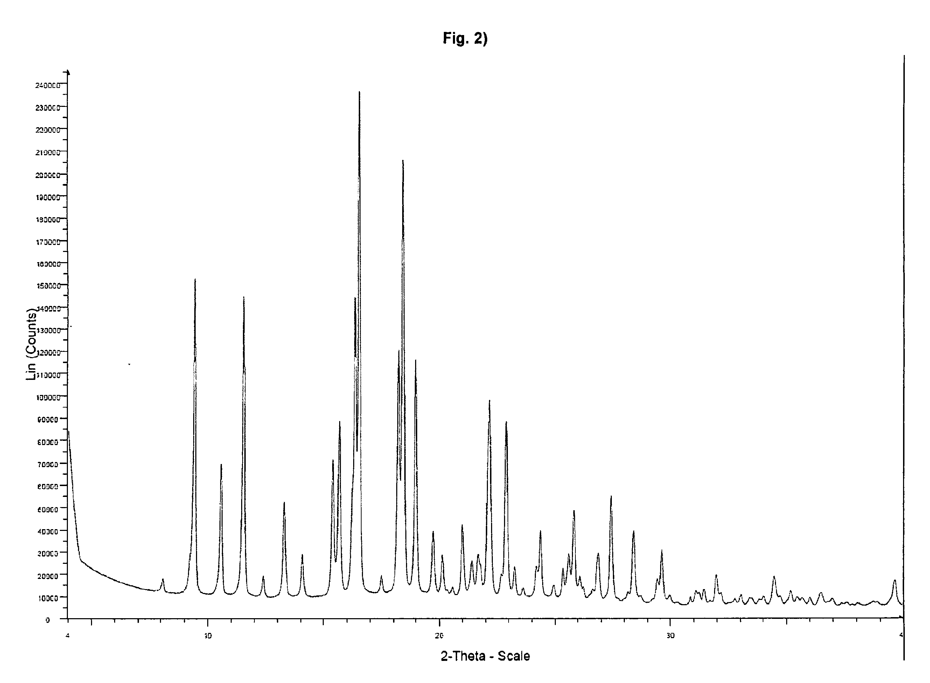

### Powder X-Ray diffraction pattern of (+)-tramadol-diflunisal crystalline salt form "A" (XRPD) (Fig. 2)

Powder diffraction patterns were acquired on a D8 Advance Series 2Theta/Theta powder diffraction system using Cu_{Kα}-radiation in transmission geometry. The system is equipped with a V 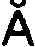 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0.

**Table 1.- List of selected peaks obtained by powder X-ray diffraction of (+)-tramadol / diflunisal crystalline salt form "A" (see figure 2).**

| Angle 2θ¹ | d value | Relative | | Angle 2θ¹ | d value | Relative |
|---|---|---|---|---|---|---|
| (°) | (Å) | Intensity % | | (°) | (Å) | Intensity % |
| 8,036 | 10,99346 | 7,4 | | 19,719 | 4,49857 | 16,5 |
| 9,406 | 9,39467 | 64,5 | | 20,121 | 4,40964 | 12,1 |
| 10,554 | 8,37529 | 29,1 | | 20,559 | 4,31657 | 5,8 |
| 11,526 | 7,67134 | 61,1 | | 20,994 | 4,22806 | 17,7 |
| 12,364 | 7,15303 | 7,9 | | 21,417 | 4,14566 | 10,9 |
| 13,290 | 6,65657 | 22,1 | | 21,701 | 4,09188 | 12,1 |
| 14,072 | 6,28869 | 12,1 | | 22,157 | 4,00883 | 40,2 |
| 15,378 | 5,75745 | 30,1 | | 22,896 | 3,88109 | 37,3 |
| 15,658 | 5,65504 | 37,4 | | 23,244 | 3,82368 | 9,8 |
| 16,322 | 5,42624 | 60,9 | | 23,626 | 3,76279 | 5,6 |
| 16,516 | 5,36303 | 100,0 | | 24,185 | 3,67708 | 9,9 |
| 17,488 | 5,06711 | 8,1 | | 24,365 | 3,65024 | 16,6 |
| 18,213 | 4,86701 | 50,9 | | 24,939 | 3,56754 | 6,2 |
| 18,430 | 4,81027 | 87,1 | | 25,368 | 3,50811 | 9,4 |
| 18,967 | 4,67521 | 49,1 | | 25,603 | 3,47653 | 12,2 |

| | | | | | | |
|---|---|---|---|---|---|---|
| ¹The 2θ values were obtained using copper radiation (CuKα1 1.54060A) | | | | | | |

### Example 2: (+)-tramadol-diflunisal crystaline salt form "B".

### Preparation:

A solution of diflunisal (20 mg, 0.08 mmol, 1 eq) in 0.5 mL of methanol was added to a solution of (+)-tramadol (21 mg, 0.08 mmol, 1 eq) in 0.5 mL of methanol. The mixture was evaporated to dryness, and the residue obtained was slurried in diisopropyl ether (3 mL). The resulting suspension was filtered off, washed with diisopropyl ether and dried under vacuum (10 mm Hg) at 40°C for 6 hours to give an abundant white solid corresponding to the salt (+)-tramadol-diflunisal of crystalline form "B".

The (+)-tramadol-diflunisal crystalline salt form "B" has been obtained starting with a ratio of (+)-tramadol / diflunisal from 2:1 to 1:2.

### Alternative Preparations:

The salt has been also obtained by evaporation at low temperature in ethanol (ratios 2:1 to 1:2), THF (ratio 1:1) and ethyl acetate (ratio 1:1), and by cooling crystallization in toluene (ratios 1:1 and 2:1) and in water/ethanol mixture (ratios 2:1 and 1:2).

### Characterization of (+)-tramadol-diflunisal crystalline salt form "B":

### ¹H-NMR spectrum of the (+)-tramadol-diflunisal crystalline salt form "B"

Proton nuclear magnetic resonance analyses were recorded in deuterated methanol (MeOH-d4) in a Bruker Avance 400 Ultrashield NMR spectrometer. Spectra were acquired solving 2 - 10 mg of sample in 0.4 - 0.8 mL of deuterated solvent.
**¹H NMR** (400 MHz, *d4*-methanol) δ: 1.45-2 (m, 8H), 2.22 (m, 1H), 2.62 (m, 1H), 2.64 (s, 6H), 2.95 (dd, *J* = 9 Hz, *J* = 13 Hz, 1H), 3.81 (s, 3H), 6.85 (m, 2H), 6.95-7.15 (m, 4H), 7.30 (t, *J* = 8 Hz, 1H), 7.47 (m, 2H), 8.01 (s, 1H).

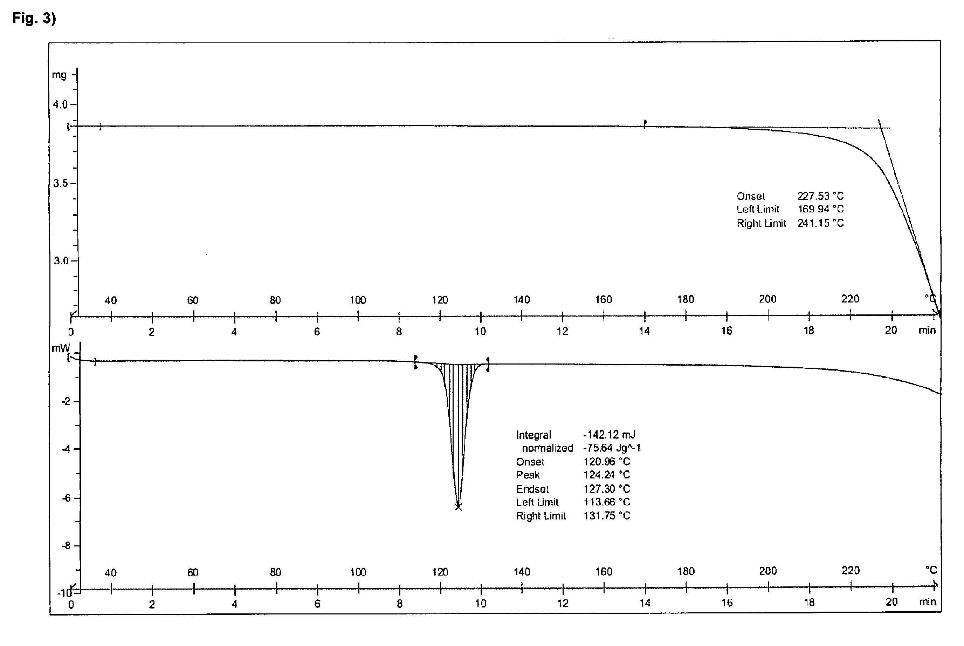

### DSC analysis of the (+)-tramadol-diflunisal crystalline salt form "B" (see Fig. 3)

DSC analyses were recorded in a Mettler Toledo DSC822e. Samples of 1-2 mg were weighted into 40 µL aluminium crucibles with a pinhole lid, and were heated, under nitrogen (50 mL/min), at 10°C/min from 30 to 300°C.
**DSC** (10°C/min): Endothermic peak corresponding to the melting point with an onset at 121 °C (see figure 3).

### TG analysis of the (+)-tramadol-diflunisal crystalline salt form "B" (see Fig. 3)

Thermogravimetric analyses were recorded in a Mettler Toledo SDTA851e. Samples of 3 - 4 mg were weighted into 40µL aluminium crucibles with a pinhole lid, and heated at 10°C/min from 30 to 600°C, under nitrogen (80 mL/min).
**TG** (10°C/min): No weight loss observed at temperatures below the melting point (see figure 3).

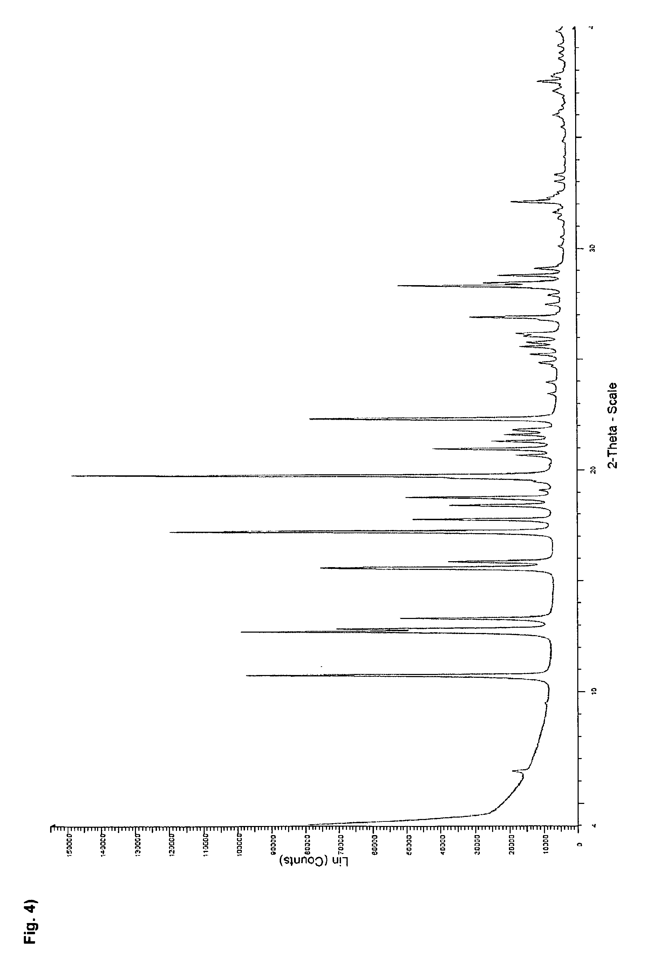

### Powder X-Ray diffraction pattern of (+)-tramadol-diflunisal crystalline salt form "B" (XRPD) (Fig. 4)

Powder diffraction patterns were acquired on a D8 Advance Series 2Theta/Theta powder diffraction system using Cu_{Kα}-radiation in transmission geometry. The system is equipped with a V 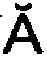 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0.

**Table 2. List of selected peaks obtained by powder X-Ray diffraction of salt (+)-tramadol-diflunisal crystalline form "B" (see figure 4).**

| Angle (2θ)¹ | d-value (Å) | Relative intensity % | | Angle (2θ)¹ | d-value (Å) | Relative intensity % |
|---|---|---|---|---|---|---|
| 10,752 | 8,22144 | 50,9 | | 21,839 | 4,06637 | 14,2 |
| 12,718 | 6,95457 | 70,1 | | 22,366 | 3,97180 | 46,0 |
| 12,825 | 6,89716 | 45,5 | | 24,898 | 3,57331 | 12,8 |
| 13,325 | 6,63945 | 32,3 | | 25,275 | 3,52089 | 10,9 |
| 15,593 | 5,67854 | 46,7 | | 25,628 | 3,47309 | 16,8 |
| 15,886 | 5,57443 | 27,7 | | 25,807 | 3,44946 | 12,7 |
| 17,236 | 5,14068 | 97,9 | | 26,112 | 3,40991 | 14,0 |
| 17,803 | 4,97818 | 40,6 | | 26,202 | 3,39833 | 13,0 |
| 18,435 | 4,80882 | 25,8 | | 26,974 | 3,30283 | 24,5 |
| 18,795 | 4,71767 | 31,0 | | 27,533 | 3,23706 | 8,6 |
| 19,798 | 4,48082 | 100,0 | | 28,373 | 3,14306 | 33,3 |
| 20,702 | 4,28715 | 27,5 | | 28,521 | 3,12706 | 21,0 |
| 21,350 | 4,15852 | 16,3 | | 28,861 | 3,09101 | 16,2 |
| 21,662 | 4,09925 | 15,0 | | | | |

| | | | | | | |
|---|---|---|---|---|---|---|
| ¹The 2θ values were obtained using copper radiation (Cu_{Kαa1} 1.54060Å) | | | | | | |

### Example 3: (-)-tramadol-diflunisal crystalline salt form "A"

### Preparation

A solution of diflunisal (20 mg, 0.08 mmol, 1 eq) in 0.5 mL of methanol was added to a solution of (-)-tramadol (21 mg, 0.08 mmol, 1 eq) in 0.5 mL of methanol. The mixture was evaporated to dryness, and the residue obtained was dissolved in acetone (0.3 mL). The resulting solution was evaporated at low temperature (-18°C). After two weeks, a solid was formed, which was dried under vacuum (10 mm Hg) at 40°C for 6 hours to give an abundant white solid corresponding to the salt (-)-tramadol / diflunisal of crystalline form "A".

The (-)-tramadol / diflunisal salt of crystalline form "A" has been obtained starting with a ratio of (-)-tramadol / diflunisal of 1:1. The salt has been also obtained by evaporation at low temperature in chloroform and tetrahydrofurane.

### Characterization of (-)-tramadol / diflunisal crystalline salt form "A"

### ¹H-NMR spectrum of the (-)-tramadol-diflunisal crystalline salt form "A"

Proton nuclear magnetic resonance analyses were recorded in deuterated methanol (MeOH-d4) in a Bruker Avance 400 Ultrashield NMR spectrometer. Spectra were acquired solving 2 - 10 mg of sample in 0.4 - 0.8 mL of deuterated solvent.
**¹H NMR** (400 MHz, d4-methanol) δ: 1.45-2 (m, 8H), 2.22 (m, 1H), 2.62 (m, 1H), 2.64 (s, 6H), 2.95 (dd, *J* = 9 Hz, *J* = 13 Hz, 1H), 3.81 (s, 3H), 6.85 (m, 2H), 6.95-7.15 (m, 4H), 7.30 (t, *J* = 8 Hz, 1H), 7.47 (m, 2H), 8.01 (s, 1H).

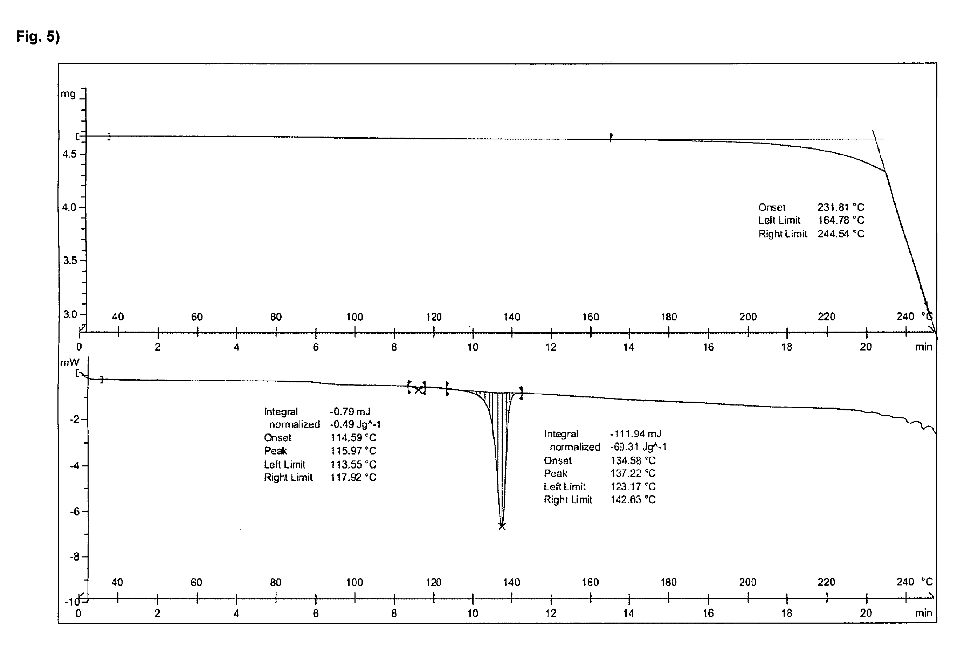

### DSC analysis of the (-)-tramadol-diflunisal crystalline salt form "A" (see Fig. 5)

DSC analyses were recorded in a Mettler Toledo DSC822e. Samples of 1-2 mg were weighted into 40 µL aluminium crucibles with a pinhole lid, and were heated, under nitrogen (50 mL/min), at 10°C/min from 30 to 300°C.
DSC (10°C/min): Endothermic peak corresponding to the melting point with an onset at 135°C.

### TG analysis of the (-)-tramadol-diflunisal crystalline salt form "A" (see Fig. 5)

Thermogravimetric analyses were recorded in a Mettler Toledo SDTA851e. Samples of 3 - 4 mg were weighted into 40 µL aluminium crucibles with a pinhole lid, and heated at 10°C/min from 30 to 600°C, under nitrogen (80 mL/min).
TG (10°C/min): No weight loss observed at temperatures below the melting point.

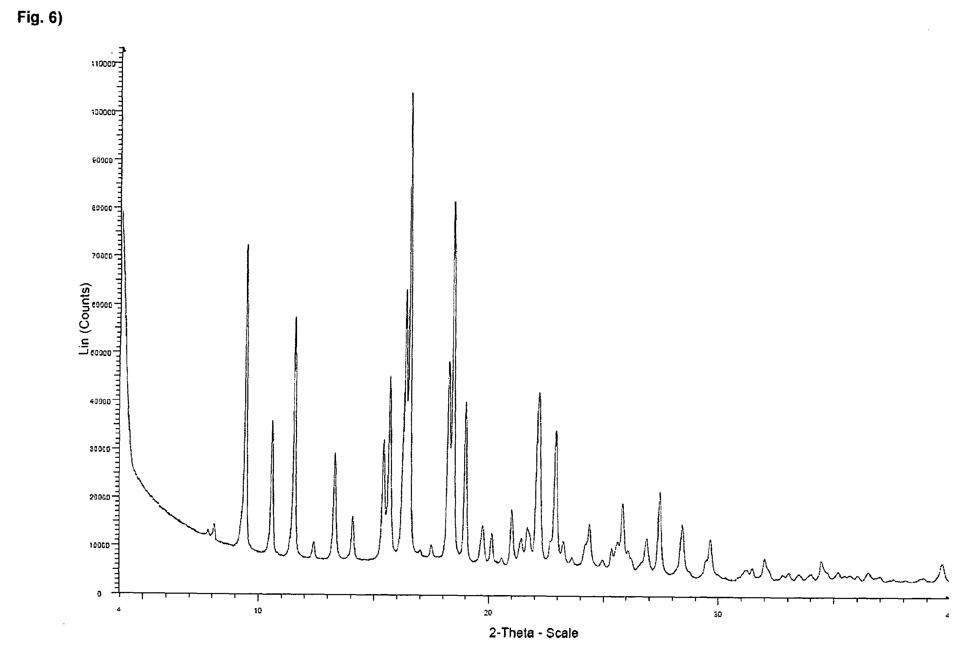

### Powder X-Ray diffraction pattern of (-)-tramadol-diflunisal crystalline salt form "A" (XRPD) (Fig. 6)

Powder diffraction patterns were acquired on a D8 Advance Series 2Theta/Theta powder diffraction system using Cu_{Kα}-radiation in transmission geometry. The system is equipped with a V 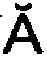 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0.

**Table 3.- List of selected peaks obtained by powder X-ray diffraction of salt (-)-tramadol / diflunisal of crystalline form "A" (see figure 6).**

| Angle 2θ¹ | d value (Å) | Relative | | Angle 2θ¹ | d value | Relative |
|---|---|---|---|---|---|---|
| (°) | | Intensity % | | (°) | (Å) | Intensity % |
| 7,788 | 11,34221 | 12,6 | | 18,431 | 4,80998 | 78,2 |
| 8,054 | 10,96842 | 13,8 | | 18,966 | 4,67554 | 38,5 |
| 9,415 | 9,38567 | 69,4 | | 19,718 | 4,49870 | 13,7 |
| 10,570 | 8,36273 | 34,4 | | 20,117 | 4,41050 | 12,2 |
| 11,538 | 7,66316 | 55,2 | | 20,567 | 4,31493 | 7,3 |
| 12,372 | 7,14879 | 10,2 | | 21,005 | 4,22587 | 17,1 |
| 13,289 | 6,65722 | 28,0 | | 21,406 | 4,14777 | 11,3 |
| 14,068 | 6,29009 | 15,4 | | 21,711 | 4,09008 | 13,5 |
| 15,405 | 5,74715 | 30,8 | | 22,161 | 4,00799 | 40,3 |
| 15,670 | 5,65069 | 43,5 | | 22,910 | 3,87868 | 32,8 |
| 16,333 | 5,42259 | 60,8 | | 23,251 | 3,82256 | 10,7 |
| 16,520 | 5,36167 | 100,0 | | 23,624 | 3,76313 | 7,5 |
| 16,993 | 5,21364 | 8,8 | | 24,390 | 3,64651 | 14,3 |
| 17,493 | 5,06576 | 9,9 | | 24,955 | 3,56535 | 7,0 |
| 18,245 | 4,85856 | 46,4 | | 25,371 | 3,50780 | 9,5 |

| | | | | | | |
|---|---|---|---|---|---|---|
| ¹The 2θ values were obtained using copper radiation (CuKα1 1.54060A) | | | | | | |

### Example 4: (-)-tramadol-diflunisal crystalline salt form "B"

### Preparation:

A solution of diflunisal (20 mg, 0.08 mmol, 1 eq) in 0.5 mL of methanol was added to a solution of (-)-tramadol (21 mg, 0.08 mmol, 1 eq) in 0.5 mL of methanol. The mixture was evaporated to dryness, and the residue obtained was slurried in diisopropyl ether (3 mL). The resulting suspension was filtered off, washed with diisopropyl ether and dried under vacuum (10 mm Hg) at 40°C for 6 hours to give an abundant white solid corresponding to the salt (-)-tramadol-diflunisal form "B".

The (-)-tramadol-diflunisal salt form "B" has been obtained starting with a ratio of (-)-tramadol/diflunisal from 2:1 to 1:2.

### Alternative Preparations:

The salt has been also obtained by evaporation at low temperature in ethanol (ratio 2:1), and by evaporation at room temperature in toluene (ratios 1:1 and 2:1).

### Characterization of (-)-tramadol-diflunisal crystalline salt form "B":

### ¹H-NMR spectrum of the (-)-tramadol-diflunisal crystalline salt form "B"

Proton nuclear magnetic resonance analyses were recorded in deuterated methanol (MeOH-d4) in a Bruker Avance 400 Ultrashield NMR spectrometer. Spectra were acquired solving 2 - 10 mg of sample in 0.4 - 0.8 mL of deuterated solvent.
**¹H NMR** (400 MHz, d4-methanol) δ: 1.45-2 (m, 8H), 2.22 (m, 1H), 2.62 (m, 1H), 2.64 (s, 6H), 2.95 (dd, *J* = 9 Hz, *J* = 13 Hz, 1H), 3.81 (s, 3H), 6.85 (m, 2H), 6.95-7.15 (m, 4H), 7.30 (t, i= 8 Hz, 1 H), 7.47 (m, 2H), 8.01 (s, 1H).

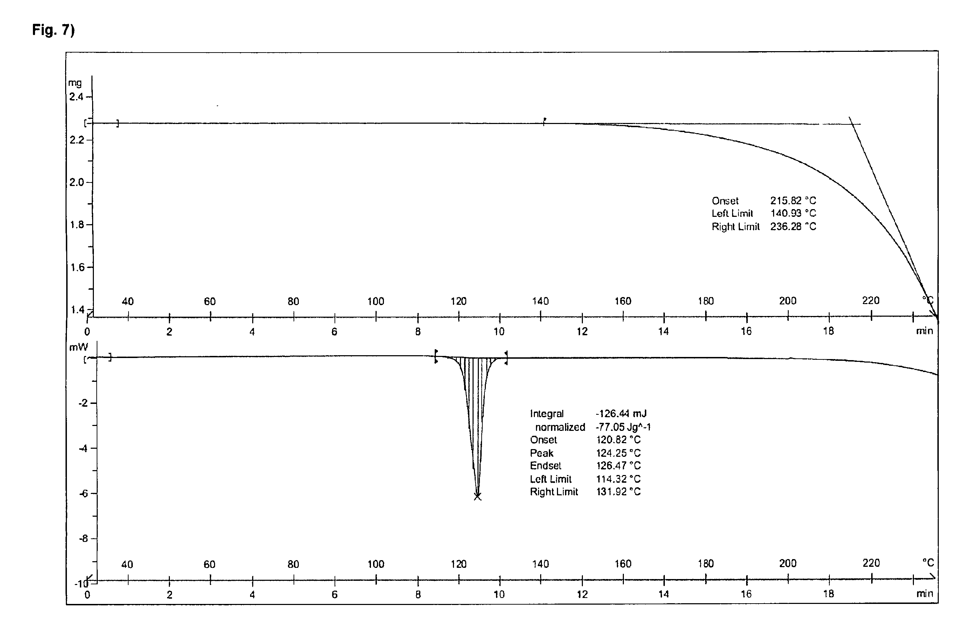

### DSC analysis of the (-)-tramadol-diflunisal crystalline salt form "B" (see Fig. 7)

DSC analyses were recorded in a Mettler Toledo DSC822e. Samples of 1-2 mg were weighted into 40 µL aluminium crucibles with a pinhole lid, and were heated, under nitrogen (50 mL/min), at 10°C/min from 30 to 300°C.
**DSC** (10°C/min): Endothermic peak corresponding to the melting point with an onset at 121 °C (see figure 7).

### TG analysis of the (-)-tramadol-diflunisal crystalline salt form "B" (see Fig. 7)

Thermogravimetric analyses were recorded in a Mettler Toledo SDTA851e. Samples of 3 - 4 mg were weighted into 40 µL aluminium crucibles with a pinhole lid, and heated at 10°C/min from 30 to 600°C, under nitrogen (80 mL/min).
TG (10°C/min): No weight loss observed at temperatures below the melting point (see figure 7).

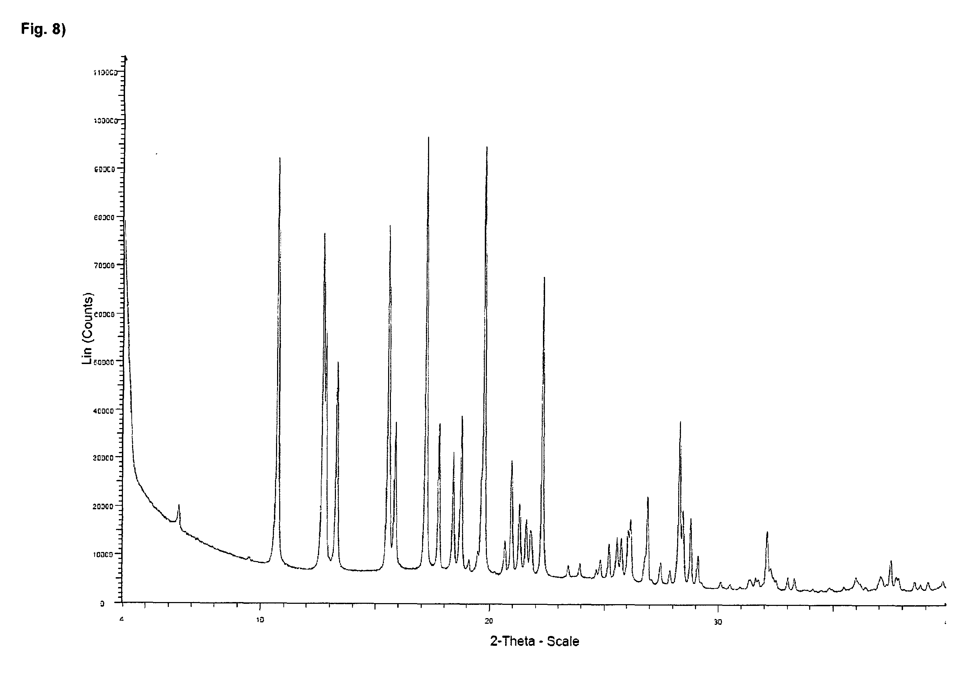

### Powder X-Ray diffraction pattern of (-)-tramadol-diflunisal crystalline salt form "B" (XRPD) (Fig. 8)

Powder diffraction patterns were acquired on a D8 Advance Series 2Theta/Theta powder diffraction system using Cu_{Kα}-radiation in transmission geometry. The system is equipped with a V 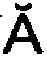 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0.

**Table 4. List of selected peaks obtained by powder X-Ray diffraction of salt (-)-tramadol-diflunisal of crystalline form "B" (see figure 8).**

| Angle (2θ)¹ | d-value (Å) | Relative intensity % | | Angle (2θ)¹ | d-value (Å) | Relative intensity % |
|---|---|---|---|---|---|---|
| 10,749 | 8,22400 | 95,1 | | 22,359 | 3,97308 | 82,4 |
| 12,724 | 6,95147 | 91,3 | | 23,539 | 3,77647 | 15,1 |
| 13,320 | 6,64196 | 61,3 | | 24,006 | 3,70410 | 13,1 |
| 15,583 | 5,68205 | 86,6 | | 25,266 | 3,52212 | 20,9 |
| 15,878 | 5,57706 | 47,1 | | 25,625 | 3,47360 | 19,9 |
| 17,238 | 5,14010 | 97,2 | | 25,812 | 3,44875 | 18,1 |
| 17,801 | 4,97869 | 46,0 | | 26,168 | 3,40273 | 22,8 |
| 18,435 | 4,80881 | 38,1 | | 26,973 | 3,30289 | 27,9 |
| 18,788 | 4,71941 | 40,4 | | 27,527 | 3,23766 | 15,6 |
| 19,798 | 4,48086 | 100,0 | | 27,972 | 3,18720 | 10,9 |
| 20,713 | 4,28483 | 18,8 | | 28,369 | 3,14347 | 50,0 |
| 20,997 | 4,22760 | 42,6 | | 28,531 | 3,12605 | 25,9 |
| 21,340 | 4,16030 | 31,5 | | 28,861 | 3,09102 | 23,4 |
| 21,642 | 4,10291 | 25,7 | | 29,181 | 3,05785 | 17,2 |
| 21,852 | 4,06409 | 24,4 | | 32,205 | 2,77728 | 23,5 |
| ¹The 2θ values were obtained using copper radiation (Cu_{Kα1} 1.54060Å) | | | | | | |

### Example 5: (rac)-tramadol-diflunisal crystalline salt

### Preparation:

A solution of diflunisal (20 mg, 0.08 mmol, 1 eq) in 0.5 mL of methanol was added to a solution of (rac)-tramadol (21 mg, 0.08 mmol, 1 eq) in 0.5 mL of methanol. The mixture was evaporated to dryness, and the residue obtained was slurried in diisopropyl ether (3 mL). The resulting suspension was filtered off, washed with diisopropyl ether and dried under vacuum (10 mm Hg) at 40°C for 6 hours to give an abundant white solid corresponding to the salt (rac)-tramadol-diflunisal.

The (rac)-tramadol-diflunisal salt has also been obtained starting with a ratio of (rac)-tramadol/diflunisal from 2:1 to 1:2.

### Alternative Preparations:

The salt has been also obtained by evaporation at low temperature in chloroform (ratios 1:1 to 2:1), THF (ratios 1:1 to 2:1), acetone (1:1 to 1:2) and ethyl acetate (ratio 1:1), by evaporation at room temperature in methyl ethyl ketone (ratios 1:1 to 2:1), and by cooling crystallization in isopropanol (ratios 1:1 to 2:1), toluene (ratios 1:1 to 2:1) and an ethanol/water mixture (ratio 1:1).

### Characterization of (rac)-tramadol-diflunisal crystalline salt:

### ¹H-NMR spectrum of the (rac)-tramadol-diflunisal crystalline salt

Proton nuclear magnetic resonance analyses were recorded in deuterated methanol (MeOH-d4) in a Bruker Avance 400 Ultrashield NMR spectrometer. Spectra were acquired solving 2 - 10 mg of sample in 0.4 - 0.8 mL of deuterated solvent.
**¹H NMR** (400 MHz, *d4*-methanol) δ: 1.45-2 (m, 8H), 2.22 (m, 1H), 2.62 (m, 1H), 2.64 (s, 6H), 2.95 (dd, *J* = 9 Hz, *J* = 13 Hz, 1H), 3.81 (s, 3H), 6.85 (m, 2H), 6.95-7.15 (m, 4H), 7.30 (t, *J* = 8 Hz, 1H), 7.47 (m, 2H), 8.01 (s, 1H).

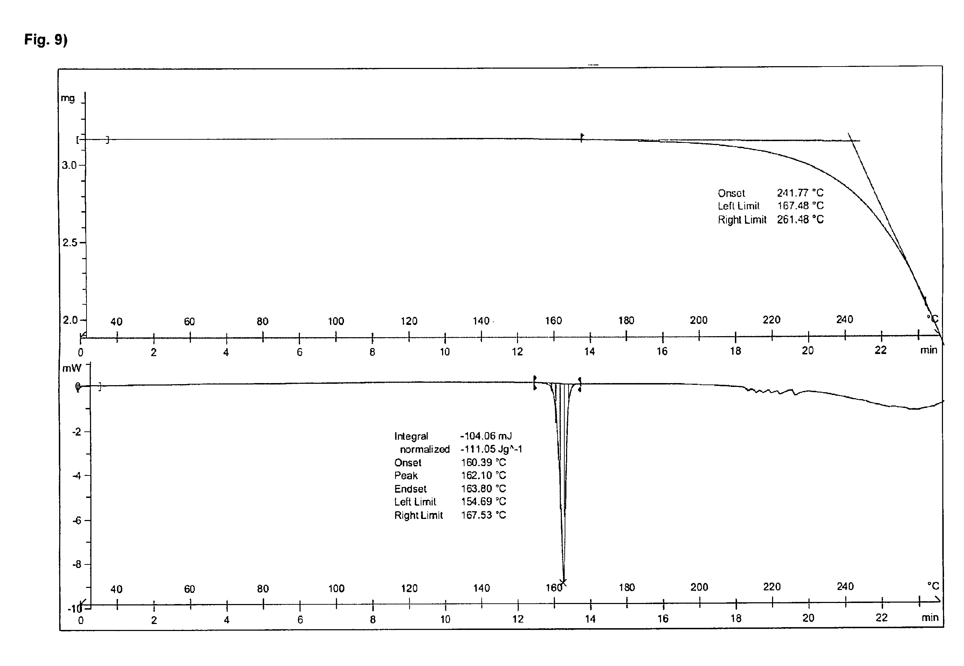

### DSC analysis of the (rac)-tramadol-diflunisal crystalline salt (see Fig. 9)

DSC analyses were recorded in a Mettler Toledo DSC822e. Samples of 1-2 mg were weighted into 40µL aluminium crucibles with a pinhole lid, and were heated, under nitrogen (50 mL/min), at 10°C/min from 30 to 300°C.
**DSC** (10°C/min): Endothermic peak corresponding to the melting point with an onset at 160°C (see figure 9)

### TG analysis of the (-)-tramadol-diflunisal crystalline salt (see Fig. 9)

Thermogravimetric analyses were recorded in a Mettler Toledo SDTA851e. Samples of 3 - 4 mg were weighted into 40 µL aluminium crucibles with a pinhole lid, and heated at 10°C/min from 30 to 600°C, under nitrogen (80 mL/min).
**TG** (10°C/min): No weight loss observed at temperatures below the melting point (see figure 9).

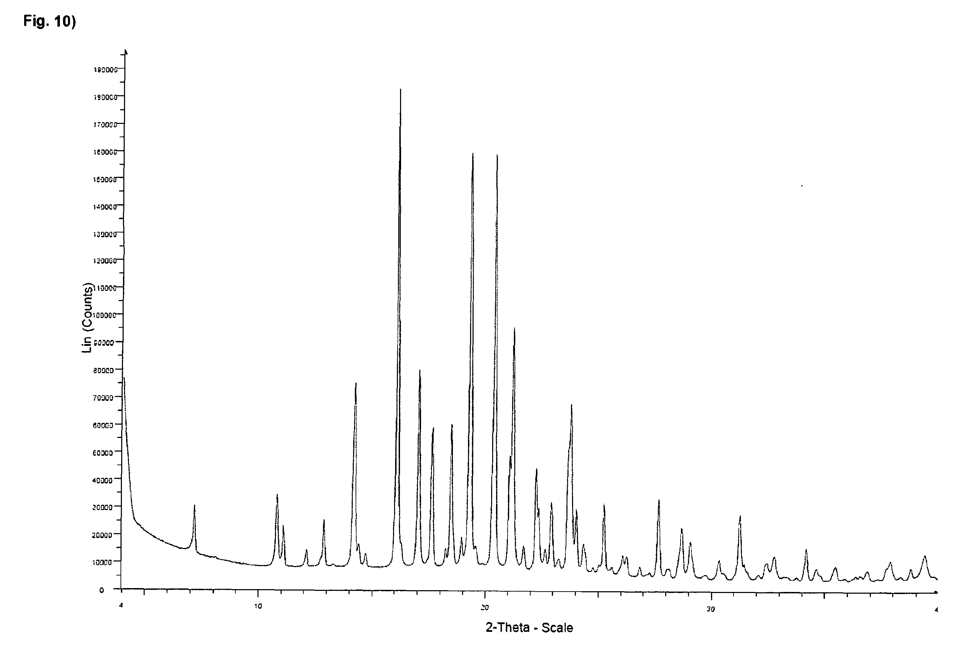

### Powder X-Ray diffraction pattern of (rac)-tramadol-diflunisal crystalline salt (XRPD) (Fig. 10)

Powder diffraction patterns were acquired on a D8 Advance Series 2Theta/Theta powder diffraction system using Cu_{Kα}-radiation in transmission geometry. The system is equipped with a V 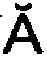 NTEC-1 single photon counting PSD, a Germanium monochromator, a ninety positions auto changer sample stage, fixed divergence slits and radial soller. Programs used: Data collection with DIFFRAC plus XRD Commander V.2.4.1 and evaluation with EVA V.12.0.

**Table 5. List of selected peaks obtained by powder X-Ray diffraction of the crystalline salt (rac)-tramadol-diflunisal (see figure 10)**

| Angle (2θ)¹ | d-value (Å) | Relative intensity % | | Angle (2θ)¹ | d-value (Å) | Relative intensity % |
|---|---|---|---|---|---|---|
| 4,130 | 21,37632 | 73,0 | | 21,242 | 4,17939 | 44,0 |
| 7,188 | 12,28786 | 19,5 | | 21,710 | 4,09033 | 9,7 |
| 10,817 | 8,17218 | 24,4 | | 22,282 | 3,98650 | 27,5 |
| 11,093 | 7,96937 | 15,6 | | 22,410 | 3,96403 | 16,6 |
| 12,100 | 7,30861 | 10,9 | | 22,673 | 3,91876 | 10,3 |
| 12,892 | 6,86145 | 14,9 | | 22,955 | 3,87121 | 22,1 |
| 14,230 | 6,21903 | 42,4 | | 23,812 | 3,73377 | 42,7 |
| 14,418 | 6,13845 | 11,2 | | 24,055 | 3,69656 | 21,0 |
| 14,726 | 6,01072 | 9,3 | | 24,385 | 3,64729 | 13,2 |
| 16,125 | 5,49223 | 100,0 | | 25,298 | 3,51765 | 22,4 |
| 17,066 | 5,19152 | 50,1 | | 26,115 | 3,40951 | 9,2 |
| 17,667 | 5,01621 | 32,3 | | 26,308 | 3,38495 | 9,9 |
| 18,297 | 4,84484 | 10,2 | | 26,911 | 3,31038 | 7,9 |
| 18,500 | 4,79211 | 29,6 | | 27,716 | 3,21609 | 25,6 |
| 18,973 | 4,67374 | 13,4 | | 28,737 | 3,10411 | 18,5 |
| 19,339 | 4,58609 | 83,5 | | 29,122 | 3,06386 | 11,6 |
| 20,430 | 4,34348 | 82,7 | | 30,388 | 2,93909 | 9,5 |
| 21,108 | 4,20554 | 30,5 | | 31,316 | 2,85407 | 19,1 |

| | | | | | | |
|---|---|---|---|---|---|---|
| ¹The 2θ values were obtained using copper radiation (Cu_{Kα1} 1.54060Å) | | | | | | |

## Claims

1. A salt of tramadol with diflunisal.

2. The salt according to claim 1 formed of (rac)-tramadol with diflunisal

3. The salt according to claim 1 formed of (+)-tramadol with diflunisal

4. The salt according to claim 1 formed of (-)-tramadol with diflunisal.

5. Crystalline form of a salt according to any of claims 1 to 4.

6. Crystalline form of a salt according to claim 2, **characterized in that** it shows an X-Ray powder diffraction pattern with peaks [2θ] at 4.1, 7.2, 10.8, 11.1, 12.1, 12.9, 14.2, 14.4, 14.7, 16.1, 17.1, 17.7, 18.3, 18.5, 19.0, 19.3, 20.4, 21.1, 21.2, 21.7, 22.3, 22.4, 22.7, 23.0, 23.8, 24.0,24.4, 25.3, 26.1, 26.3, 26.9, 27.7, 28.7, 29.1, 30.4 and 31.3 [°]. The 2θ values were obtained using copper radiation (Cu_{Kα1} 1.54060Å).

7. Crystalline form of a salt according to claim 2, **characterized in that** the endothermic peak corresponding to the melting point has an onset at 160°C.

8. Crystalline form "A" of a salt according to claim 3, **characterized in that** it shows an X-Ray powder diffraction pattern with peaks [2θ] at 8.0, 9.4, 10.6, 11.5, 12.4, 13.3, 14.1, 15.4, 15.7, 16.3, 16.5, 17.5, 18.2, 18.4, 19.0, 19.7, 20.1, 20.5, 21.0, 21.4, 21.7, 22.2, 22.9, 23.2, 23.6, 24.2, 24.4, 24.9, 25.4 and 25.6 [°].

9. Crystalline form "A" of a salt according to claim 3, **characterized in that** the endothermic peak corresponding to the melting point has an onset at 135°C.

10. Crystalline form "B" of a salt according to claim 3, **characterized in that** it shows an X-Ray powder diffraction pattern with peaks [2θ] at 10.8, 12.7, 12.8, 13.3, 15.6, 15.9, 17.2, 17.8, 18.4, 18.8, 19.8, 20.7, 21.4, 21.7, 21.8, 22.4, 24.9, 25.3, 25.6, 25.8, 26.1, 26.2, 27.0, 27.5, 28.4, 28.5, and 28.9 [°].The 26 values were obtained using copper radiation (Cu_{Kα1} 1.54060Å).

11. Crystalline form "B" of a salt according to claim 3, **characterized in that** the endothermic peak corresponding to the melting point has an onset at 121 °C.

12. Crystalline form "A" of a salt according to claim 4, **characterized in that** it shows an X-Ray powder diffraction pattern with peaks [2θ] at 7.8, 8.1, 9.4, 10.6, 11.5, 12.4, 13.3, 14.1, 15.4, 15.7, 16.3, 16.5, 17.0, 17.5, 18.2, 18.4, 19.0, 19.7, 20.1, 20.6, 21.0, 21.4, 21.7, 22.1, 22.9, 23.2, 23.6, 24.4, 24.9 and 25.4 [°].

13. Crystalline form "A" of a salt according to claim 4, **characterized in that** the endothermic peak corresponding to the melting point has an onset at 135°C.

14. Crystalline form "B" of a salt according to claim 4, **characterized in that** it shows an X-Ray powder diffraction pattern with peaks [2θ] at 10.7, 12.7, 13.3, 15.6, 15.9, 17.2, 17.8, 18.4, 18.8, 19.8, 20.7, 21.0, 21.3, 21.6, 21.9, 22.4, 23.5, 24.0, 25.3, 25.6, 25.8, 26.2, 27.0, 27.5, 28.0, 28.4, 28.5, 28.9, 29.2, and 32.2 [°]. The 2θ values were obtained using copper radiation (Cu_{Kα1} 1.54060Å).

15. Crystalline form "B" of a salt according to claim 4, **characterized in that** the endothermic peak corresponding to the melting point has an onset at 121 °C.

16. Process for the production of a salt according to claim 1 comprising the steps of:
a) dissolving diflunisal as a free acid in an organic solvent and either together, before or after dissolving tramadol either as a free base or as a salt in an organic solvent, leading to a mixture in which both active principles are dissolved in one or more organic solvents;
b) evaporating the solvent to dryness,
and either
c1) forming a slurry of the residue of step b) in a second organic solvent;
e1) filtering, drying and/or purifying the resulting product;
or
c2) dissolving the residue of step b) in a second organic solvent:
d2) evaporating the second organic solvent at low temperature;
e2) filtering, drying and/or purifying the resulting product.

17. Process according to claim 16, wherein
• the organic solvent of step a) is methanol; and/or
• the second organic solvent of step c1) is diisopropyl ether; and/or
• the second organic solvent of step c2) is selected from acetone, ethanol, THF, ethyl acetate, chloroform; preferably acetone; and/or
• the temperature for evaporating the second organic solvent in step d2) is - 18°C; and/or
• the ratio of tramadol to diflunisal is 2:1 to 1:2.

18. Medicament comprising at least one salt according to any of claims 1 to 4 and optionally one or more pharmaceutically acceptable excipients.

19. Pharmaceutical composition **characterized in that** it comprises a therapeutically effective amount of the salt according to any one of claims 1 to 4 or the crystalline form of a salt according to any one of claims 5 to 15, in a physiologically acceptable medium.

20. Use of a salt according to any one of claims 1 to 4 or the crystalline form of a salt according to any one of claims 5 to 15 for the treatment of pain, preferably acute pain, chronic pain, neuropathic pain, hyperalgesia, allodynia or cancer pain, including diabetic neuropathy and osteoarthritis.
